# EUROPEAN PATENT SPECIFICATION

(11) **EP 0 914 313 B1**
(45) Date of publication and mention of the grant of the patent: **24.07.2002**
(21) Application number: 97916536.2
(22) Date of filing: 11.04.1997
(51) Int. Cl.: C07C 43/11, C11D 1/72

(54) **NON-IONIC SURFACTANT COMPOSITIONS**
NICHTIONISCHE TENSIDMISCHUNGEN
COMPOSITIONS TENSIOACTIVES NON IONIQUES

(30) Priority: 16.04.1996 GB 9607865
(43) Date of publication of application: 12.05.1999
(73) Proprietor: IMPERIAL CHEMICAL INDUSTRIES PLC, London SW1P 3JF (GB)
(72) Inventor: DANIELS, Judith, Yarm, Cleveland TS15 9LF (GB); THOMAS, Hugh, Middlesbrough, Cleveland TS9 6DS (GB)
(74) Representative: Roberts, Jonathan Winstanley
(86) International application number: GB9700999
(87) International publication number: WO9738961

(56) References cited:
- DE-A- 2 257 642
- FR-A- 2 303 850
- GB-A- 1 462 134
- US-A- 3 691 081
- US-A- 4 129 514

## Description

This invention relates to non-ionic surfactant, particularly detergent, compositions and in particular to surfactant, and especially detergent, compositions of or containing combinations of alcohol ethoxylates.

Alcohol ethoxylates, are widely used non-ionic surfactants, for example in domestic detergents such as dish wash liquids and laundry cleaning products, both liquid and powder. The properties of these materials vary with structure and the group is a flexible and very useful class of detergent type surfactant. Similarly, the ecological behaviour and toxicological properties of these surfactants vary, but in general they have low mammalian toxicity and are more or less readily broken down in the environment to non-toxic products. However, the standards applied to detergent materials are becoming increasingly strict, particularly on aquatic toxicity and biodegradability.

In relation to aquatic toxicity, effective detergency in a basically aqueous environment is likely to generate some toxicity for aquatic organisms because the effects of the detergents and their tendency to collect at oil/water interfaces, including their effect on interfacial tension, makes them likely to interfere with the breathing mechanisms of aquatic organisms. To meet current and future standards, detergent product manufacturers are looking to use less toxic materials in their formulations. For example, under European Union Law e.g. European Commission Directive 67/548/EEC, if manufacturers are to avoid having to label their products as being or containing materials that are "Dangerous to the Environment", strict limits are placed on the aquatic toxicity of detergent formulations and the materials that go into them. To avoid this designation, the requirements include an assessment of the effects of the detergents on a range of aquatic taxa, but particularly sensitive species include *Daphnia magna.* In practice, in tests on *Daphnia magna,* the EC₅₀ (the test concentration which immobilises 50% of the test organisms), on 48 hour exposure has to be more than 1 mg.litre⁻¹. Unfortunately, the effectiveness of alcohol ethoxylates, as detergents, especially at the lower temperatures that are becoming more usual particularly in domestic laundry applications, is generally inversely related to their aquatic toxicity, almost certainly due in part to the interfacial effects noted above.

The problem is made more severe with the tendency to use lower temperatures, particularly in laundry cleaning, than have been used previously. The effective use of lower temperatures requires detergents that have good detergency at the relatively low use temperatures. This has caused detergent formulation manufacturers to use alcohol ethoxylates which have relatively low cloud points, and thus relatively short ethoxylate chains. Such detergents have relatively high aquatic toxicity. Detergents based on block copolymers of ethylene oxide (EO) and propylene oxide (PO) can give good detergency with low toxicity, but these compounds have relatively poor ultimate biodegradability, which limits their use.

We have investigated the relationship between toxicity and structure in alcohol ethoxylates. Conventional synthetic routes to such ethoxylates give a spread of ethoxylate chain lengths typically including significant proportions of non-, mono- and di-ethoxylated alcohols, collectively referred to as "low ethoxylates". Our experimental findings indicate that a significant contribution to toxicity is made by low ethoxylates of relatively long carbon chain e.g. C₁₃ to C₁₅, alcohols, particularly where the average length of the polyethoxylate chain is relatively short e.g. less than about 5 residues. The movement to lower laundry temperatures has generally increased the proportion of such relatively toxic compounds in detergents because alcohol polyethoxylates having 3 to 5 residues with C₁₃ to C₁₅ alkyl chains have cloud points in the appropriate range to make them effective detergents at such lower temperatures. We believe that these compounds contribute to toxicity disproportionately to their concentration in the ethoxylates as manufactured. Increasing the length of the ethoxylate chain will reduce the toxicity, both because the proportion of low ethoxylates is reduced and the addition of (further) ethoxylate residues dilutes the low ethoxylates. However, dilution alone does not fully account for the reduction in toxicity that we have observed, as simple bulk dilution with relatively non-toxic ethoxylates is less effective than would be expected on this basis. As the conventional relatively non-toxic ethoxylates are relatively short chain materials they are less effective detergents and the fall off in overall surfactancy/detergency appears to be greater than the reduction in toxicity.

Other commonly used surfactants include alkyl phenol ethoxylates e.g. nonyl phenol ethoxylates. However, in recent years, pressures for the replacement of this class of surfactant has grown because it has been suggested that they do not biodegrade sufficiently rapidly and because breakdown products may exhibit toxicity or oestrogenic activity in the environment.

The present invention is based on our finding that certain combinations of alcohol ethoxylates can provide good detergency and relatively low toxicity without impairing the good biodegradability of standard alcohol ethoxylates. In particular, preferred materials have toxicity which is low enough that that they do not need to be classified as "Dangerous to the Environment" on the definition outlined above. The low toxicity of the materials of the invention is such that they may also be attractive substitutes for other surfactants e.g. alkyl phenol ethoxylates, where there are environmental pressures for replacement.

Accordingly the present invention provides an alcohol ethoxylate surfactant composition, particularly a detergent composition, of compounds of the formula (la):

HO . (EO)ₙ₁ . R¹ (Ia)

and of the formula (lb):

HO . (EO)ₙ₂ . R² (Ib)

in which:
- EO: is an ethylene oxide residue (-CH₂.CH₂.O-);
- n1: is from 3 to 8; and
- n2: is from 4.5 to 9;
- R¹: is alkyl selected from alkyl groups having from 8 to 12 carbon atoms and having an average number of carbon atoms from 9 up to less than 12; and
- R²: is alkyl selected from alkyl groups having from 12 to 16 carbon atoms and having an average number of carbon atoms from 12 to 15;
in weight proportions such that the overall average number of carbon atoms in the alkyl groups R¹ and R² is from 10.5 to 12 and the overall average number of EO units is from 4.5 to 8.
In particular the invention provides an alcohol ethoxylate surfactant composition, particularly a detergent composition, in which the alcohol ethoxylates are of the formula (II):

HO . (EO)ₙ₃ . R³ (II)

in which:
- EO: is an ethylene oxide residue (-CH₂.CH₂.O-);
- R³: is alkyl from two populations R^{3a} and R^{3b}, where:
in population R^{3a}, the alkyl groups are selected from alkyl groups having from 8 to 12 carbon atoms and have an average number of carbon atoms from 9 up to less than 12; and
in population R^{3b}, the alkyl groups are selected from alkyl groups having from 12 to 16 carbon atoms and have an average number of carbon atoms from 12 to 15;
- n3: is from two populations n3^{a} and n3^{b}, corresponding to the populations R^{3a} and R^{3b} respectively, where n3^{a} is from 3 to 8 and n3^{b} is from 4.5 to 9;
where the weight proportions of the two populations is such that the overall average number of carbon atoms in the alkyl groups of R³ is from 10.5 to 12 and the overall average value of n3 is from 4.5 to 8.

In a particularly desirable embodiment that forms a separate and specific aspect, the invention provides a surfactant composition, particularly a detergent composition, including a mixed-alcohol ethoxylate of the formula (III):

HO . (EO)ₙ₄ . R⁴ (III)

in which:
- EO: is an ethylene oxide residue (-CH₂.CH₂.O-);
- n4: is from 4.5 to 8; and
- R⁴: is the alkyl residues of a mixed-alcohol such that:
a first portion of the alkyl groups is selected from alkyl groups having from 8 to 12 carbon atoms and having an average number of carbon atoms from 9 up to less than 12; and
a second portion of the alkyl groups is selected from alkyl groups having from 12 to 16 carbon atoms and having an average number of carbon atoms from 12 to 15;
the respective alcohols being present in the mixed-alcohol in weight proportions such that the overall average number of carbon atoms in the alkyl groups of R⁴ is from 10.5 to 12.

In this aspect of the invention the term "mixed-alcohol ethoxylate" means an alcohol ethoxylate made by ethoxylating a mixture of alcohols, sometimes referred to as "mixed-alcohol" (in terms of alcohols R⁴OH, where R⁴ is as defined in Formula (III)) in which the carbon numbers of the alkyl groups of the alcohols meets the requirements for the alkyl groups in the ethoxylates set out above. Such mixed-alcohols can be made by:
1 mixing appropriate alcohols, especially by mixing alcohols which individually satisfy the alkyl groups in the ethoxylates set out above
2 synthesis, for example by carbonylating e.g. using the OXO process, a mixture of olefins having a corresponding composition (allowing that the OXO olefin starting material has one less carbon atom per molecule that the product alcohol) to that of the alkyl groups in the ethoxylates set out above.

The invention includes a method of making the compositions of the invention which includes ethoxylating a mixed-alcohol having an average alkyl chain length as defined above so as to form a mixed-alcohol ethoxylate having an average number of EO units of from 4.5 to 8.

Using mixed-alcohols as a starting material has the particular advantage that the mixed-alcohol ethoxylate is the direct product of the ethoxylation process. This benefits users of the mixed-alcohol ethoxylate because, as the product has not been made by blending pre-formed ethoxylates, it is not regarded as an intentional mixture of the (notional) individual ethoxylates for the purpose of considering ecological significance e.g. aquatic toxicity as described above.

The mixed-alcohol ethoxylate can be made by:
1 direct reaction of mixed-alcohol and ethylene oxide usually in the presence of a catalyst, such as a base e.g. alkoxide generated *in situ* by the reaction between the alcohol and KOH on removal of water, or
2 etherification of the mixed-alcohols and pre-formed polyethylene glycol.
The most convenient route will usually be to react the mixed-alcohols with ethylene oxide. Corresponding reactions are routinely used to make conventional ethoxylates on an industrial scale and are generally well known in the art. An further option is to use so-called narrow range ethoxylation conditions to produce an ethoxylate having a narrower distribution of polyoxyethylene chain lengths about the average chain length. This will generally reduce the proportion of high carbon number alcohol and the corresponding low ethoxylates in the product surfactant/detergent composition (see also below) and this may give a further reduction in the toxicity of the product.

Narrow range ethoxylation processes differ from the conventional processes outlined above by using special catalysts.

Within the above ranges, the invention is particularly applicable to ethoxylates, especially of mixed-alcohols, where the overall average number of carbon atoms (*C*) in the alkyl residues of the composition is from about 10.5 to 11.5 more particularly 10.75 to 11.25 and especially about 11.0. In the component alcohols, especially component mixed-alcohols, the alcohol having an average carbon chain length of less than 12 desirably has an average carbon chain length of from about 9 to about 11 and the alcohol having an average carbon chain length of 12 or more desirably has an average chain length of from about 12 to about 15, particularly from 12 to 14.5.

The alkyl groups used in the compositions of the invention will often be derived from distillation cuts and/or natural sources. As such, they will include a spread of alkyl chain lengths and may include small proportions of alkyl groups lying outside the ranges given above for the alkyl groups [from 8 to 12 for the groups having an average number of carbon atoms from 9 up to less than 12; and from 12 to 16 for the groups having an average number of carbon atoms from 12 to 15]. The proportion of alkyl groups having chain lengths outside the stated ranges for the two types of alkyl groups is less than 10% by weight, is desirably less than 5% by weight and typically not more than about 2% by weight (based on the corresponding alcohols).

From a toxicity standpoint, it is desirable to minimise the amount of alcohol residue having high carbon numbers, particularly to minimise the amount of the corresponding low ethoxylates, and to maximise the weight proportion of ethylene oxide in the molecule. However, the use of unduly high proportions of relatively short chain alcohols or unduly long polyethylene oxide chains tends to give products with inferior detergent performance. These considerations flow through to the weight proportions of the component ethoxylates used in the composition so that the relative proportions of the component ethoxylates are typically (expressed as weight percentages of the basic alcohols) at least 50%, more usually at least about 60%, desirably at least about 65%, particularly at least about 70%, and especially about 75%, up to about 85%, particularly about 80%, of alcohol having an average carbon chain length of less than 12. These correspond to ranges of from about 50 to about 85%, desirably from about 65 to about 80%, particularly from about 70 to about 80% and especially from about 75 to about 80% of alcohol having an average carbon chain length of less than 12 and correspondingly from about 50 to about 15%, desirably from about 35 to about 20%, particularly from about 30 to about 20% and especially from about 25 to about 20% of alcohol having an average carbon chain length of 12 or more.

The polyethoxylate residues in the molecules of the alcohol ethoxylate detergent composition of the invention have an overall average length (*EO*) of from 4.5 to 9, desirably from about 5 to about 7. Overall, the use of shorter average ethylene oxide chains can lead to undesirably high proportions of unreacted alcohol or low (poly)ethoxylates with the overall composition being more toxic than is desirable. The use of longer ethylene oxide chains can make the detergent too soluble in water and reduce its effectiveness in aqueous detergency applications, particularly in emulsifying fatty and oily soils at relatively low wash temperatures. As it is desirable to minimise the proportion of the relatively short chain ethoxylates based on the alcohol having an average carbon chain length of 12 or more, *EO* will usually satisfy the relationship: *EO* >= *C* - 6; corresponding to a value of *EO* of at least 6 when *C* = 12 and *EO* of at least 4.5 when *C* = 10.5.

Where the composition is a mixed-alcohol ethoxylate, the distribution of polyethoxylate chain lengths is determined by the reaction conditions used to ethoxylate the mixed-alcohol, so that *EO* = n4 [in formula (III)]. Where the composition is made by mixing pre-formed ethoxylates the polyethoxylate chain lengths are determined by the reaction conditions used to ethoxylate the individual alcohols and can thus vary between the ethoxylate based on the alcohol having an average carbon chain length of less than 12 and that based on the alcohol having an average carbon chain length of more than 12. In this case it is desirable that the average number of ethylene oxide residues in the ethoxylate based on the alcohol having an average carbon chain length of 12 or more is equal to or greater than the average number of ethylene oxide residues in the ethoxylate based on the alcohol having an average carbon chain length of less than 12. Conveniently, the average number of ethylene oxide residues in the ethoxylates is the same, within close limits. However, where the average ethoxylate chain lengths are different, it is also desirable that the difference is not more than about 3 and desirably 2 repeat ethylene oxide residues.

Among mixed-alcohol ethoxylate compositions of the formula (III), particularly useful compositions are those of the formula (IIIa): HO.(EO)ₙ₄.R⁴ where EO, n4, and R⁴ are generally as defined in formula (III) above such that the overall average alkyl chain length (*C*) is 10.75 to 11.25 and the overall average ethylene oxide chain length (*EO*) is 5 to 8, particularly 5 to 6. Such materials can have good wash performance and relatively low toxicity. Also useful are compositions of the formula (IIIb): HO.(EO)ₙ₄.R⁴ where EO, n4, and R⁴ are generally as defined in formula (III) above such that the overall average alkyl chain length (*C*) is 11.25 to 12 and the overall average ethylene oxide chain length (*EO*) satisfies the relationship: *EO* = *C* - 5.5 and desirably satisfies the relationship: *EO* = *C* - 5 which can have low toxicity and adequate wash performance. The wash performance of such materials is not as good as those of formula (IIIa) especially at relatively lower wash temperatures e.g. about 40°C, but at higher wash temperatures e.g. about 60°C is typically similar to that of the compounds of the formula (IIIa).

Particularly useful compositions of the general formula (III) are those where the weight proportion of from about 65% to about 80%, particularly about 75% to about 80% of alcohol having an average carbon chain length of less than 12 and from about 35% to about 20%, particularly about 25% to about 20%, of alcohol having an average carbon chain length of 12 or more such that the overall average alkyl chain length (*C*) is 10.75 to 11.25 and the overall average ethylene oxide chain length (*EO*) is 5 to 8, particularly 5 to 6. Where the component alcohols are typical detergent alcohols having an average of about 10 and about 13.7 for the alcohol having an average carbon chain length of less than 12 and the alcohol having an average carbon chain length of 12 or more respectively, compositions within these weight ranges approximately correspond to compositions of the formula (IIIa) above.

Among mixed alcohol ethoxylate compositions of the formula (I) or (II), particularly useful compositions are those where the overall average alkyl chain length (*C*) is 10.75 to 11.25 and the overall average ethylene oxide chain length (*EO*) is 5 to 8, particularly 5 to 6. Such materials can have good wash performance and relatively low toxicity.

The surfactant and detergent compositions of this invention are useful in a variety of end use applications including general purpose detergency, including laundry and hard surface cleaner applications, in fabric and fibre scouring and as emulsifiers. In some applications the compositions of the invention will generally substitute for other alcohol alkoxylates, particularly ethoxylates, which have relatively high aquatic toxicity and in some other applications, the compositions of the invention may substitute for other surfactants such as alkylphenol alkoxylates, particularly alkylphenol ethoxylates e.g. nonylphenol ethoxylates (NPE's), and particularly such ethoxylates containing an average of from 3 to 20 units of EO residue per molecule. Alkylphenol alkoxylates are no longer typically used in domestic detergent applications in western Europe but are used elsewhere and in other detergent applications such as institutional hard surface cleaning, and in fibre, yarn and fabric scouring and in applications that may not be usually considered as detergent applications such as in emulsification applications.

The invention accordingly includes detergent formulations including the alcohol ethoxylate compositions of the invention, methods of cleaning using detergent formulations including the alcohol ethoxylate compositions of the invention and the use of the alcohol ethoxylate compositions of the invention as detergents. The invention further includes emulsifier formulations including the alcohol ethoxylate compositions of the invention, methods of emulsification using surfactant formulations including the alcohol ethoxylate compositions of the invention and the use of the alcohol ethoxylate compositions of the invention as emulsifiers.

Laundry detergents can be considered in two groups, domestic laundry and industrial and institutional laundry products. The formulations used are broadly similar, the main differences are that domestic laundry products will typically include components such as fragrances that are often not included in industrial/institutional laundry formulations.

In domestic laundry applications, the surfactant and detergent compositions of this invention will typically be used to replace non-ionic, particularly other alcohol ethoxylate (or other alkoxylate) detergents. Suitable general detergent formulations are well known in the art and the compositions of this invention can replace alcohol ethoxylates in those applications where they are currently used. Typical broadly stated formulations, which are conventional apart from the use of the surfactant/detergent compositions of this invention, for powder laundry detergents, aqueous liquid laundry detergents and non-aqueous liquid laundry detergents are described below.

Powder laundry detergents are typically available as "traditional" or "conventional" powders or as "concentrated" powders. The main difference is that concentrated powders include less filler (and the formulation is re-balanced to take account of this). The main components are detergent surfactants, usually including non-ionic detergents, such as alcohol ethoxylates, in this invention replaced by the surfactant/detergent compositions of the invention, and anionic detergents, such as linear alkylbenzene sulphonates, alkane sulphates, fatty alcohol sulphates, alcohol ethoxysulphates and fatty acid soaps; builders which may be non-phosphate builders such as zeolites e.g. zeolite A, or sodium carbonate, or phosphate builders, particularly condensed phosphate builders such as sodium tripolyphosphate and tetrapostassium pyrophosphate; and auxiliaries such as bleaches, particularly peroxygen bleaches such as perborates, and bleach activators; enzymes such as proteases, liapses, amylases and cellulases; optical brighteners; co-builders such as polycarboxylates and sodium citrate; antiredeposition aids such as sodium carboxymethylcellulose (CMC); corrosion inhibitors such as sodium silicate; fillers such as sodium sulphate; and hydrotropes such as sodium xylene sulphonate and sodium toluene sulphonate. Typical amounts are as follows:

| Material | amount (% by weight) | |
|---|---|---|
| | conventional | concentrated |
| Non-ionic detergent (of the invention) | 1 to 5 | 3 to 15 |
| Anionic detergent | 8 to 25 | 5 to 35 |
| Builder/co-builder | 25 to 50 | 25 to 60 |
| Bleach and activator | 0 to 25 | 0 to 25 |
| filler | 5 to 35 | 0 to 12 |
| other additives | 0 to 7 | 1 to 10 |

Aqueous liquid laundry detergents are presented as water base solutions of the formulation components. The formulation components are generally similar to those used in powders, but fillers such as sodium sulphate are not used, the builder (if used) is typically a citrate builder (in solution) and is used at a relatively low level and bleaches are not included because they are not stable in the presence of the water base. The base may include small amounts of solvents other than water, such as lower alcohols. Typical proportions for conventional and concentrated aqueous liquid laundry detergents are as follows:

| Material | amount (% by weight) | |
|---|---|---|
| | conventional | concentrated |
| Non-ionic detergent (of the invention) | 1 to 10 | 1 to 30 |
| Anionic detergent | 5 to 25 | 10 to 35 |
| Builder/co-builder | 3 to 30 | 0 to 36 |
| other additives | 0.1 to 5 | 1 to 5 |
| water and other solvents | 65 to 75 | 28 to 56 |

Non-aqueous liquid laundry detergents are typically dispersions of solids, usually mainly the builder, is a liquid phase, which is usually mainly the detergents and may include polyalkylene glycols, especially polyethylelene glycol. Generally they are relatively more concentrated in detergents and builders that aqueous liquids and typical proportions for non-aqueous liquid laundry detergents are as follows:

| Material | amount (% by weight) |
|---|---|
| Non-ionic detergent (of the invention) | 3 to 30 |
| Other detergents (including anionics)* | 0 to 12 |
| ilder/co-builder | 25 to 60 |
| CMC | 0.5 to 5 |
| bleach and bleach activator | 0 to 20 |
| other additives (fragrance, enzymes etc.) | 0 to 5 |
| polyalkylenen glycol | 0 to 50 |

The other surfactants may include anionic surfactants if desired, but particularly where the builder is a hydroxycarboxylic acid salt such as citrate or tartrate, the surfactant will often include amine oxide surfactants (see e.g. WO 92/20772 A). The surfactant/detergent compositions of the invention are particularly useful in non-aqueous liquid laundry detergents especially of the type described in our earlier patent specifications EP 0030096 B, EP 0120659 B, EP 0346113 B, WO 92/20772 A and WO 94/03580 A.

Industrial and institutional laundry products typically have compositions falling broadly within the above indicated ranges, except that materials such as fragrances are often not included and strong alkali may be included to improve the cleaning action particularly with heavily soiled materials. For convenience of use, particularly metering into wash systems, liquid detergents are particularly useful in industrial and institutional laundry products. The availability of mechanised and automated metering makes it practical to use multi-pack detergent systems so that the concentrations used and relative concentrations of sub-systems can be controlled by the user to suit the particular circumstances e.g. to vary the amount of builder depending on the wash water hardness. It also makes it practical to use substantial amounts of caustic alkali even though alcohol ethoxylates are not hydrolytically stable to aqueous strong/caustic alkali, because the alkali can be included in a pack with the builder separate from the surfactants. Examples of typical multi-pack systems using the surfactant/detergent compositions of the invention are given below:

| Material | amount (% by weight) | | | | |
|---|---|---|---|---|---|
| | Type A | | Type B | | |
| Pack A | | | | | |
| Non-ionic detergent (of the invention) | 20 | | 20 | | |
| CMC | 1 | | 1 | | |
| water to | 100 | | 100 | | |

| Pack B | | | | | |
|---|---|---|---|---|---|
| sodium silicate | 5 | 9.5 | - | - | - |
| sodium metasilicate | - | 16.8 | 22.5 | 19.5 | 26.4 |
| tetrapostassium pyrophosphate | 10 | 9.5 | - | 9 | - |
| potassium hydroxide | 14 | 30.4 | - | - | 13.5 |
| potassium carbonate | - | - | 14.5 | 11.5 | 7.6 |
| water to | 100 | 100 | 100 | 100 | 100 |
| mix ratio Pack A:Pack B | 1:4 | 1:2 | 1:2 | 1:2 | 1:2 |

If required a third pack can be used to provide a bleach and (if used) a bleach activator in suitable amounts.

The invention therefore specifically includes laundry detergent formulations including the alcohol ethoxylate detergent compositions of the invention, in particular solid detergent granules, aqueous liquid formulations, especially heavy duty laundry liquid formulations and non-aqueous liquid formulations, especially heavy duty laundry liquid formulations, particularly heavy duty non-aqueous laundry liquid formulations; methods of cleaning laundry using detergent formulations including the alcohol ethoxylate detergent compositions of the invention and the use of the alcohol ethoxylate detergent compositions of the invention as detergents in laundry washing.

The cleaning of hard surfaces, particularly metals, ceramics, glass and plastics, is carried out in domestic, institutional and industrial environments. The surfactant/detergent compositions of this invention, being alcohol ethoxylates, do not have sufficient alkali stability for use in heavy duty industrial cleaning and degreasing applications where highly alkaline washing media are used (to provide effective cleaning/degreasing with a minimum of mechanical scrubbing). However, they can be highly effective in general hard surface cleaning applications as detergents and wetting agents. Typical hard surface cleaners in which the surfactant/detergent compositions of this invention can be used are as follows:

| Material | amount (% by weight) |
|---|---|
| Surfactant (including surfactant of the invention) | 0.5 to 25% |
| Builder | 0 to 30 % |
| Chelating agent | 0 to 20% |
| Alkali | 0 to 40 % |
| Solvent | 0 to 20% |
| Dye, fragrance, preservative etc. | 0 to 2 |
| Water | Balance |

This generic formulation can be used for both domestic and industrial/institutional applications. Similarly to laundry products industrial/institutional hard surface cleaners can include strong/caustic alkali, usually in relatively small amounts e.g. up to about 5% of the total formulation. The use of alkali and bleaches can be simplified in industrial/institutional applications by the use of multi-pack systems similar to the way these are used in laundry applications.

The invention therefore specifically includes hard surface cleaner detergent formulations including the alcohol ethoxylate detergent compositions of the invention; methods of cleaning hard surfaces using detergent formulations including the alcohol ethoxylate detergent compositions of the invention and the use of the alcohol ethoxylate detergent compositions of the invention as detergents in hard surface cleaning.

The surfactant compositions of the invention can be used in fibre, yam and fabric scouring applications. Such end uses include scouring fabrics, particularly natural fabrics such as cotton to clean them after weaving and synthetic fabrics especially to remove materials that have been applied to the fibre or fabric to improve processing e.g. spin finish compositions and textile auxiliaries. Scouring can also be carried out on raw natural fibres to remove materials that detract from the value or ease of processing of the fibre in spinning and/or weaving; an example of this is wool scouring. As sheared from the sheep, the woollen fleece includes, in addition to the wool fibre, a variety of materials including wool wax. Wool wax is a complex mixture of fatty materials secreted onto the wool by the skin of the sheep. Wool wax is removed from the wool, prior to use of the wool in textiles, by washing the wool with an aqueous mix including surfactants typically at moderately elevated temperatures e.g. 40 to 80°C, particularly 50 to 70°C - and this process is called wool scouring. The surfactant compositions of this invention can be used as replacements for NPE surfactants in wool scouring, including in methods using multiple scouring cycles. In this application the surfactant composition of the invention is typically used at a concentration of from 0.1 to 5, particularly from 0.2 to 1.5, g.l⁻¹ and typically in an amount of 0.25 to 2% by weight based on the dry weight of the greasy wool. The products from wool scouring are clean wool and the recovered wool fat.

The invention therefore specifically includes fibre, yarn and fabric scouring formulations including the alcohol ethoxylate detergent compositions of the invention; methods of scouring fibres, yarns and fabrics using detergent formulations including the alcohol ethoxylate detergent compositions of the invention and the use of the alcohol ethoxylate detergent compositions of the invention as detergents in scouring .

Fibre, yam and fabric scouring can be considered as a cleaning process on the fibre, yarn or fabric and an emulsification process on the material removed from the fabric or fibre, especially where this can be a valuable side or recovered product. The surfactant compositions of this invention are also useful in emulsification applications which would not normally be considered as detergent applications. Thus they can be used to emulsify oils in water for example in oil in water emulsion spin finish and textile auxiliary applications. The surfactant compositions of this invention typically have HLB values in the range 10 to 14, more usually 11 to 13, and are thus particularly applicable to the emulsification of oils which have an HLB requirement in these ranges. In emulsification applications of this kind, the proportion of surfactant composition will typically be from 0.1 to 3%, more usually 0.5 to 1% based on the weight of the emulsion.

The invention therefore specifically includes emulsion formulations including the alcohol ethoxylate detergent compositions of the invention; methods of emulsification of oils in water, particularly oils having an HLB requirement in the range 10 to 14, using surfactant formulations including the alcohol ethoxylate surfactant compositions of the invention and the use of the alcohol ethoxylate detergent compositions of the invention as emulsifiers, particularly in making oil in water emulsions, particularly of oils having an HLB requirement in the range 10 to 14.

The following Examples illustrate the invention. All parts and percentages are by weight unless otherwise indicated.

### Materials used

- Acropol: C13/15 mixed alcohols *ex* Exxon
- Dobanol: C9/11 mixed alcohols *ex* Shell
- C12/14: C12/14 mixed alcohols (nominally lauric acid) *ex* Henkel or Proctor & Gamble
- Mycol 1095: (>95%) C10 linear alcohol from natural fat/oil sources *ex Kao*
- Mycol 2098: A C12/14 linear alcohol from natural fat/oil sources *ex* Kao
- S91/2.5: Synperonic 91/2.5 a C9/11 alcohol (2.5) ethoxylate *ex* ICI Surfactants
- S91/5: Synperonic 91/5 a C9/11 alcohol (5) ethoxylate *ex* ICI Surfactants
- S91/6: Synperonic 91/6 a C9/11 alcohol (6) ethoxylate *ex* ICI Surfactants
- S91/8: Synperonic 91/8 a C9/11 alcohol (8) ethoxylate *ex* ICI Surfactants
- SA3: Synperonic A3 a C13/15 alcohol (3) ethoxylate *ex* ICI Surfactants
- SA4: Synperonic A4 a C13/15 alcohol (4) ethoxylate *ex* ICI Surfactants
- SA5: Synperonic A5 a C13/15 alcohol (5) ethoxylate *ex* ICI Surfactants
- SA7: Synperonic A7 a C13/15 alcohol (7) ethoxylate *ex* ICI Surfactants
- SA3/87K: a 1:2 wt. mix of Synperonic A3 and Synperonic 87K [C13/15 alcohol (6) ethoxylate including a small proportion of propylene oxide units *ex* ICI Surfactants] - a conventional non-ionic blend having good laundry cleaning properties - used as a cleaning standard.
- Silcolapse: silicone antifoam
- Tinopal CBS-X: optical brightener *ex* Ciba Geigy
- Emiphos STP-8: sodium tripolyphosphate builder *ex* Albright and Wilson
- Wessalith: zeolite builder *ex* Degussa

Mixed alcohol ethoxylates were made by mixing the starting alcohols in the desired weight proportions and ethoxylating the mixture using a basic catalyst (potassium alkoxylate made *in situ* by adding KOH to the mixed alcohols and removing water by distillation) to achieve the desired level of ethoxylation.

Toxicity Testing - was carried out by methods generally specified in Annex V of European Union Directive 67/548/EEC for assessing the toxicity of detergent compositions. Most compositions were tested for toxicity to *Daphnia magna* to assess the EC₅₀ for *D magna* on exposure of cohorts of 10 or 20 individuals for 48 hours . The effect observed is the immobilisation of individual *Daphnia* in a cohort. Some compositions were also tested for toxicity to Rainbow Trout (*Oncorhyncus mykiss*) and Algae (*Selenastrum capricornutum*) as the test species. Wash Testing - was carried out using a Tergometer B 10 G machine (United States Testing Company) with water of standard hardness (selected from 50, 100 and 300 ppm) at 40 or 60°C and using 10 g.l⁻¹ of formulation. Each wash used a combination of standard test soiled cloths selected from EMPA cotton 101 (101), EMPA polycotton 104 (104), Krefeld cotton 10C (10C), Krefeld polycotton 20C (20C) and EMPA 116 (116). The reflectance of the cloths was measured before and after washing and the percentage increase in reflectance is reported as the test result.

### Examples 1 to 15

Various ethoxylate mixtures were formulated either by mixing pre-made ethoxylates (mixed ethoxylates abbreviated MA) or by ethoxylating mixtures of alcohols (mixed-alcohol ethoxylates abbreviated MAA). All the formulations (apart from comparative materials) used an alcohol, Alcohol 1, having an average chain length of less than 12 carbon atoms and an alcohol, Alcohol 2 having an average chain length of more than 12 carbon atoms. The composition of the formulations is summarised in Table 1 below. The weight proportions are of ethoxylates for MA formulations and of alcohols for MAA formulations. The average carbon chain length values given in Table 1 allow for the molecular weight of the ethoxylates used. Table 1 includes toxicity testing results (EC₅₀ for *D magna* for 48 hour exposure).

The compositions of Examples 7 and 9 were subjected to further toxicity testing using *O mykiss* (Rainbow Trout) and *S capricornutum* (Algae) as the test species. The results are set out in Table 2 below (which also includes the *D magna* data from Table 1).

**Table 1**

| Ex No | Type | Alcohol 1 | | Alcohol 2 | | Ave C No | EO No* | EC₅₀ (mg.l⁻¹) |
|---|---|---|---|---|---|---|---|---|
| | | Material | (%) | Material | (%) | | | |
| C1 | MA | SA3/87K | | | | 13.6 | 4.5 | 0.4** |
| C2 | MA | S91/2.5 | 25 | SA7 | 75 | 12.2 | 5.9 | 0.7 |
| C3 | MA | S91/6 | 75 | SA3 | 25 | 11.1 | 5.3 | 0.4 |
| C4 | MAA | Dobanol | 25 | Acropol | 75 | 12.7 | 5.9 | 0.7 |
| C5 | MA | S91/8 | 60 | SA3 | 40 | 11.9 | 6 | 0.5 |
| C6 | MAA | Dobanol | 60 | Acropol | 40 | 11.4 | 3 | <0.3 |
| C7 | MAA | Dobanol | 50 | C 12/14 | 50 | 11.3 | 3 | 0.4 |
| 1 | MAA | Dobanol | 75 | Acropol | 25 | 10.9 | 5.3 | 1.4 |
| 2 | MAA | Dobanol | 50 | Acropol | 50 | 11.8 | 7 | 1.8 |
| 3 | MAA | Dobanol | 50 | Acropol | 50 | 11.8 | 5 | 0.9 |
| 4 | MAA | Dobanol | 50 | C 12/14 | 50 | 11.3 | 5 | 0.9 |
| 5 | MA | S91/5 | 67 | SA5 | 33 | 11.2 | 5 | 0.8 |
| 6 | MA | S91/5 | 70 | SA5 | 30 | 11.1 | 5 | - |
| 7 | MAA | Dobanol | 70 | Acropol | 30 | 11.0 | 5 | 1.1 |
| 8 | MAA | Dobanol | 70 | Acropol | 30 | 11.0 | 6 | 1.4 |
| 9 | MAA | Dobanol | 70 | Acropol | 30 | 11.0 | 7 | 1.8 |
| 10 | MAA | Mycol 1095 | 70 | Mycol 2098 | 30 | 10.8 | 5 | 2.4 |
| 11 | MAA | Mycol 1095 | 70 | Mycol 2098 | 30 | 10.8 | 6 | 3.1 |
| 12 | MAA | Mycol 1095 | 70 | Mycol 2098 | 30 | 10.8 | 7 | 4.3 |
| 13 | MAA | Dobanol | 70 | Mycol 2098 | 30 | 10.8 | 5 | 2.0 |
| 14 | MAA | Mycol 1095 | 70 | Acropol | 30 | 11.1 | 5 | 1.1 |

| | | | | | | | | |
|---|---|---|---|---|---|---|---|---|
| * EO No for MAA materials Average EO No (Molecular weight compensated) for MA materials | | | | | | | | |
| ** Calculated value from EC₅₀ values for SA3 *ca* 0.25 and for 87k *ca* 0.5 | | | | | | | | |

**Table 2**

| Ex No | *D magna* 48hr EC50 | *O mykiss* 96hr EC50 | *S capricornutum* EC50 |
|---|---|---|---|
| 7 | 1.1 | 3.9 | 1.3 |
| 9 | 1.8 | 2.4 | 1.3 |

### Application Examples

In the Application Examples below, aqueous based detergent formulations were tested for wash performance using the basic formulation:

| Material | parts by wt |
|---|---|
| detergent | 10.0 |
| Silcolapse | 0.1 |
| sodium carboxy methyl cellulose | 0.5 |
| Tinopal CBS-X | 0.2 |
| Builder | 17.6 |
| PEG 200 | 8.0 |
| Water to | 100.0 |

### Application Example AE1

Three aqueous laundry detergent formulations were made up using Emiphos STP-8 as builder; AEC1 used the ethoxylate of Comparative Example C1, AEC2 that of Comparative Example C4 and AE1 that of Example 1. Tergometer testing was carried out at 40 and 60°C using wash water of 50,100 and 300 ppm hardness. The results are set out in Table AE1 below.

### Application Example AE2

Three aqueous laundry detergent formulations were made up using Wessalith zeolite as builder; AEC3 used the ethoxylate of Comparative Example C1, AEC4 that of Comparative Example C4 and AE2 that of Example 1. Tergometer testing was carried out at 40 and 60°C using wash water of 50, 100 and 300 ppm hardness. The results are set out in Table AE2 below.

### Application Example AE3

Three aqueous laundry detergent formulations were made up using Emiphos STP-8 as builder; AEC5 used the ethoxylate of Comparative Example C1, AE3a that of Example 5 and AE3b that of Example 6. Tergometer testing was carried out at 40 and 60°C using wash water of 100 ppm hardness. The results are set out in Table AE3 below.

**Table AE1**

| Comp No | Temp (°C) | Hard (ppm) | 10C | 101 | 20C | 104 | 116 | Total |
|---|---|---|---|---|---|---|---|---|
| AEC1 | 40 | 50 | 39.7 | 45.2 | 41.2 | 45.6 | 11.0 | 167.4 |
| | | 100 | 38.8 | 44.0 | 39.5 | 42.5 | 12.2 | 204.1 |
| | | 300 | 36.3 | 43.0 | 37.6 | 39.8 | 10.7 | 203.5 |
| | 60 | 50 | 53.3 | 47.5 | 45.6 | 46.7 | 11.0 | 202.3 |
| | | 100 | 52.9 | 46.7 | 45.5 | 46.2 | 12.2 | 175.6 |
| | | 300 | 52.7 | 47.2 | 46.3 | 45.4 | 10.7 | 172.2 |
| AEC2 | 40 | 50 | 37.4 | 45.1 | 35.0 | 46.1 | 12.0 | 163.1 |
| | | 100 | 36.7 | 44.3 | 36.5 | 43.7 | 11.0 | 214.7 |
| | | 300 | 34.5 | 43.1 | 31.5 | 42.1 | 11.9 | 213.5 |
| | 60 | 50 | 55.5 | 47.7 | 49.6 | 49.9 | 12.0 | 213.8 |
| | | 100 | 55.2 | 47.1 | 49.6 | 50.6 | 11.0 | 176.3 |
| | | 300 | 54.3 | 47.9 | 49.6 | 50.1 | 11.9 | 175.9 |
| AE1 | 40 | 50 | 37.8 | 44.2 | 37.6 | 46.1 | 10.6 | 169.8 |
| | | 100 | 38.7 | 43.5 | 38.5 | 46.4 | 8.8 | 217.7 |
| | | 300 | 38.6 | 43.2 | 35.9 | 42.8 | 9.3 | 212.4 |
| | 60 | 50 | 57.2 | 47.7 | 50.6 | 51.6 | 10.6 | 211.8 |
| | | 100 | 55.8 | 46.9 | 50.2 | 50.7 | 8.8 | 212.4 |
| | | 300 | 54.9 | 47.0 | 50.3 | 50.3 | 9.3 | 211.8 |

**Table AE2**

| Comp No | Temp (°C) | Hard (ppm) | 10C | 101 | 20C | 104 | 116 | Total |
|---|---|---|---|---|---|---|---|---|
| AEC3 | 40 | 50 | 40.3 | 42.1 | 42.4 | 35.1 | 11.0 | 123.4 |
| | | 100 | 39.4 | 39.6 | 41.3 | 35.3 | 12.2 | 219.5 |
| | | 300 | 31.9 | 23.7 | 35.9 | 21.2 | 10.7 | 222.0 |
| | 60 | 50 | 55.3 | 50.5 | 50.5 | 52.2 | 11 | 168.2 |
| | | 100 | 56.6 | 51.1 | 49.7 | 52.4 | 12.2 | 161.7 |
| | | 300 | 52.4 | 30.6 | 43.4 | 31.1 | 10.7 | 158.3 |
| AEC4 | 40 | 50 | 35.9 | 42.3 | 36.9 | 34.6 | 12.0 | 122.9 |
| | | 100 | 31.0 | 42.6 | 35.6 | 38.1 | 11.0 | 226.9 |
| | | 300 | 26.8 | 27.6 | 33.0 | 23.6 | 11.9 | 223.3 |
| | 60 | 50 | 57.4 | 52 | 51.9 | 53.6 | 12 | 199.9 |
| | | 100 | 56.1 | 51.4 | 51.1 | 53.7 | 11 | 166.0 |
| | | 300 | 55 | 39.2 | 50.3 | 43.5 | 11.9 | 159.7 |
| AE2 | 40 | 50 | 38.1 | 41.6 | 38.7 | 37.0 | 10.6 | 117.2 |
| | | 100 | 36.9 | 40.8 | 36.0 | 37.2 | 8.8 | 226.9 |
| | | 300 | 29.0 | 22.9 | 35.6 | 20.4 | 9.3 | 223.1 |
| | 60 | 50 | 58.5 | 51.5 | 52.7 | 53.6 | 10.6 | 190.1 |
| | | 100 | 57.2 | 50.7 | 52.7 | 53.7 | 8.8 | 223.1 |
| | | 300 | 55.3 | 34.2 | 52.1 | 39.2 | 9.3 | 190.1 |

**Table AE3**

| AE No | Comp No | Temp (°C) | 10C | 101 | 20C | 104 | Total |
|---|---|---|---|---|---|---|---|
| AECS | C1 | 40 | 40.1 | 51.3 | 52.8 | 48.9 | 193.1 |
| | | 60 | 53.8 | 52.3 | 53 | 46.4 | 205.5 |
| AE3a | 5 (1:2) | 40 | 37.7 | 49.4 | 50.4 | 49.8 | 187.3 |
| | | 60 | 54.9 | 52.8 | 56.5 | 49.8 | 214 |
| AE3b | 6 (3:7) | 40 | 38.9 | 51.7 | 51.1 | 49.7 | 191.4 |
| | | 60 | 54.6 | 53 | 57.3 | 50 | 214.9 |

## Claims

1. An alcohol ethoxylate surfactant composition of compounds of the formula (la):
HO . (EO)ₙ₁ . R¹ (Ia)
and of the formula (lb):
HO . (EO)ₙ₂ . R² (Ib)
in which:
EO is an ethylene oxide residue (-CH₂.CH₂.O-);
n1 is from 3 to 8; and
n2 is from 4.5 to 9;
R¹ is alkyl selected from alkyl groups having from 8 to 12 carbon atoms and having an average number of carbon atoms from 9 up to less than 12; and
R² is alkyl selected from alkyl groups having from 12 to 16 carbon atoms and having an average number of carbon atoms from 12 to 15;
in weight proportions such that the overall average number of carbon atoms (*C*) in the alkyl groups R¹ and R² is from 10.5 to 12 and the overall average number of EO units (EO) is from 4.5 to 8.

2. An alcohol ethoxylate surfactant composition in which the alcohol ethoxylates are of the formula (II):
HO . (EO)ₙ₃ . R³ (II)
in which:
EO is an ethylene oxide residue (-CH₂.CH₂.O-);
R³ is alkyl from two populations R^{3a} and R^{3b}, where:
in population R^{3a}, the alkyl groups are selected from alkyl groups having from 8 to 12 carbon atoms and have an average number of carbon atoms from 9 up to less than 12; and
in population R^{3b}, the alkyl groups are selected from alkyl groups having from 12 to
16 carbon atoms and have an average number of carbon atoms from 12 to 15;
n3 is from two populations n3^{a} and n3^{b}, corresponding to the populations R^{3a} and R^{3b} respectively, where n3^{a} is from 3 to 8 and n3^{b} is from 4.5 to 9;
where the weight proportions of the two populations is such that the overall average number of carbon atoms (*C*) in the alkyl groups of R³ is from 10.5 to 12 and the overall average value of n3 is from 4.5 to 8.

3. A surfactant composition including a mixed-alcohol ethoxylate of the formula (III):
HO . (EO)ₙ₄ . R⁴ (III)
in which:
EO is an ethylene oxide residue (-CH₂.CH₂.O-);
n4 is from 4.5 to 8; and
R⁴ is the alkyl residues of a mixed-alcohol such that:
a first portion of the alkyl groups is selected from alkyl groups having from 8 to 12 carbon atoms and having an average number of carbon atoms from 9 up to less than 12; and
a second portion of the alkyl groups is selected from alkyl groups having from 12 to 16 carbon atoms and having an average number of carbon atoms from 12 to 15;
the respective alcohols being present in the mixed-alcohol in weight proportions such that the overall average number of carbon atoms (*C*) in the alkyl groups of R⁴ is from 10.5 to 12.

4. A composition as claimed in any one of claims 1 to 3 in which the overall average number of carbon atoms (*C*) in the alkyl residues of the composition is from 10.5 to 11.5.

5. A composition as claimed in claim 4 in which *C* is from 10.75 to 11.25.

6. A composition as claimed in any one of claims 1 to 5 in which the alcohol having an average carbon chain length of less than 12 has an average carbon chain length of from about 9 to about 11.

7. A composition as claimed in any one of claims 1 to 6 in which the alcohol having an average carbon chain length of 12 or more has an average chain length of from 12 to 14.5.

8. A composition as claimed in any one of claims 1 to 7 in which the proportion of alkyl groups having chain lengths outside the stated ranges for the two types of alkyl groups is less than 10% by weight (based on the corresponding alcohols).

9. A composition as claimed in any one of claims 1 to 8 in which the polyethoxylate residues in the molecules of the alcohol ethoxylate composition have an overall average length (*EO*) of from 5 to 7.

10. A composition as claimed in any one of claims 1 to 9 in which the overall average length of the polyethoxylate residues in the molecules of the alcohol ethoxylate (*EO*) satisfies the relationship: *EO* >= *C -* 6.

11. A composition as claimed in any one of claims 1 to 10 which is a mixed-alcohol ethoxylate having an overall average alkyl chain length (*C*) of from 10.75 to 11.25 and an overall average ethylene oxide chain length (*EO*) of from 5 to 8.

12. A composition as claimed in any one of claims 1 to 11 wherein wherein the weight ratios of the groups R1 and R2, based on the respective alcohols, are in the ranges 50:50 to 85:15.

13. A composition as claimed in claim 12 wherein the weight ratios of the groups R1 and R2, based on the respective alcohols, are in the ranges 60:40 to 85:15.

14. A composition as claimed in claim 13 wherein the weight ratios of the groups R1 and R2, based on the respective alcohols, are in the ranges 70:30 to 80:20.

15. A composition as claimed in claim 14 wherein the weight ratio of the groups R1 and R2, based on the respective alcohols, is about 75:25.

16. A method of making a composition as claimed in any one of claims 1 to 15 which includes ethoxylating a mixed-alcohol having an average alkyl chain length as defined in any one of claims 1 to 15 so as to form a mixed-alcohol ethoxylate having an average number of EO units of from 4.5 to 8.

17. A laundry detergent formulation including an alcohol ethoxylate composition as claimed in any one of claims 1 to 15.

18. A laundry detergent formulation as claimed in claim 17 in the form of solid detergent granules, an aqueous liquid formulation, or a non-aqueous liquid formulations.

19. A laundry detergent formulation as claimed either claim 17 or claim 18 in the form of a multi-pack system.

20. A method of cleaning laundry using as a detergent a formulation as claimed in any one of claims 17 to 19.

21. The use of alcohol ethoxylate compositions as claimed in any one of claims 1 to 15 in any one of claims 1 to 15, or 17 to 19 as detergents in laundry washing.

22. A hard surface cleaner formulation including an alcohol ethoxylate composition as claimed in any one of claims 1 to 15.

23. A hard surface cleaner formulation as claimed in claim 22 in the form of a multi-pack system.

24. A method of cleaning hard surfaces using a formulation including an alcohol ethoxylate composition as claimed in any one of claims 1 to 15, 22 or 23.

25. The use of alcohol ethoxylate compositions as claimed in any one of claims 1 to 15 as detergents in hard surface cleaning.

26. Fibre, yarn and fabric scouring formulations including the alcohol ethoxylate composition claimed in any one of claims 1 to 15.

27. A methods of scouring fibres, yarns and fabrics using formulations including the alcohol ethoxylate composition claimed in any one of claims 1 to 15.

28. The use of an alcohol ethoxylate composition as claimed in any one of claims 1 to 15 as a surfactant in fibre, yarn or fabric scouring.

29. Emulsion formulations including the alcohol ethoxylate compositions as claimed in any one of claims 1 to 15.

30. A method of emulsifying an oil in water using an alcohol ethoxylate compositions as claimed in any one of claims 1 to 15 as an emulsifier.

31. A method as claimed in claim 30 wherein the oil has an HLB requirement in the range 10 to 14.

32. The use of an alcohol ethoxylate composition as claimed in any one of claims 1 to 15 as an emulsifier for making oil in water emulsions.

## Patentansprüche

1. Oberflächenaktive Alkoholethoxylat-Zusammensetzung aus Verbindungen
der Formel (1a) :
HO.(EO)ₙ₁.R¹ (1a)
und
der Formel (1b) :
HO.(EO)ₙ₂.R² (1b),
in welchen:
EO ein Ethylenoxidrest (-CH₂.CH₂.O-) ist
n₁ 3 bis 8 beträgt; und
n₂ 4,5 bis 9 beträgt;
R¹ ein Alkyl ist, das aus Alkylgruppen mit 8 bis 12 Kohlenstoffatomen ausgewählt ist und eine durchschnittliche Kohlenstoffatomzahl von 9 bis weniger als 12 aufweist; und
R² ein Alkyl ist, das aus Alkylgruppen mit 12 bis 16 Kohlenstoffatomen ausgewählt ist und das eine durchschnittliche Kohlenstoffatomzahl von 12 bis 15 aufweist;
in Gewichtsanteilen derart, dass die gesamte durchschnittliche Kohlenstoffatomzahl (C) in den Alkylgruppen R¹ und R² 10,5 bis 12 beträgt und die gesamte durchschnittliche Zahl an EO-Einheiten (EO) 4,5 bis 8 beträgt.

2. Oberflächenaktive Alkoholethoxylat-Zusammensetzung, in welcher die Alkoholethoxylate folgende Formel (II) besitzen:
HO. (EO)ₙ₃.R³ (II)
in welcher:
EO ein Ethylenoxidrest (-CH₂.CH₂.O-) ist
R³ ein Alkyl aus zwei Populationen R^{3a} und R^{3b} darstellt, wobei
in Population R^{3a} die Alkylgruppen aus Alkylgruppen mit 8 bis 12 Kohlenstoffatomen und mit einer durchschnittlichen Kohlenstoffatomzahl von 9 bis weniger als 12 ausgewählt sind; und
in Population R^{3b} die Alkylgruppen aus Alkylgruppen mit 12 bis 16 Kohlenstoffatomen ausgewählt sind und eine durchschnittliche Kohlenstoffatomzahl von 12 bis 15 aufweisen;
n₃ aus zwei Populationen n3^{a} und n3^{b} ist, welche jeweils den Populationen R^{3a} und R^{3b} entsprechen, wobei n3^{a} 3 bis 8 beträgt und n3^{b} 4,5 bis 9 beträgt;
wobei die Gewichtsanteile der zwei Populationen derart sind, dass die gesamte durchschnittliche Kohlenstoffatomzahl (C) in den Alkylgruppen von R³ 10,5 bis 12 beträgt und der gesamte durchschnittliche Wert von n3 4,5 bis 8 beträgt.

3. Oberflächenaktive Zusammensetzung einschließlich eines gemischten Alkoholethoxylats der folgenden Formel (III):
HO . (EO)ₙ₄ . R⁴ (III)
in welcher:
EO ein Ethylenoxidrest (-CH₂.CH₂.O-) ist
n₄ 4,5 bis 8 beträgt; und
R⁴ die Alkylreste eines gemischten Alkohols derart darstellt, dass:
ein erster Teil der Alkylgruppen aus Alkylgruppen mit 8 bis 12 Kohlenstoffatomen und mit einer durchschnittlichen Zahl von Kohlenstoffatomen von 9 bis weniger als 12 ausgewählt ist; und
ein zweiter Teil der Alkylgruppen aus Alkylgruppen mit 12 bis 16 Kohlenstoffatomen und mit einer durchschnittlichen Zahl von Kohlenstoffatomen von 12 bis 15 ausgewählt ist;
wobei die jeweiligen Alkohole in dem gemischtem Alkohol in Gewichtsanteilen derart vorhanden sind, dass die gesamte durchschnittliche Zahl an Kohlenstoffatomen (C) in den Alkylgruppen von R⁴ 10,5 bis 12 beträgt.

4. Zusammensetzung gemäß einem der Ansprüche 1 bis 3, in welcher die gesamte durchschnittliche Zahl an Kohlenstoffatomen (C) in den Alkylresten der Zusammensetzung 10,5 bis 11,5 beträgt.

5. Zusammensetzung gemäß Anspruch 4, in welcher C 10,75 bis 11,25 beträgt.

6. Zusammensetzung gemäß einem der Ansprüche 1 bis 5, in welcher der Alkohol mit einer durchschnittlichen Kohlenstoffkettenlänge von weniger als 12 eine durchschnittliche Kohlenstoffkettenlänge von ungefähr 9 bis ungefähr 11 besitzt.

7. Zusammensetzung gemäß einem der Ansprüche 1 bis 6, in welcher der Alkohol mit einer durchschnittlichen Kohlenstoffkettenlänge von 12 oder mehr eine durchschnittlichen Kettenlänge von 12 bis 14,5 besitzt.

8. Zusammensetzung gemäß einem der Ansprüche 1 bis 7, in welcher der Anteil an Alkylgruppen mit Kettenlängen außerhalb der angegebenen Bereiche für die beiden Arten von Alkylgruppen weniger als 10 Gew.-% (basierend auf den entsprechenden Alkoholen) beträgt.

9. Zusammensetzung gemäß einem der Ansprüche 1 bis 8, in welcher die Polyethoxylatreste in den Molekülen der Alkoholethoxylatzusammensetzung eine durchschnittliche Gesamtlänge (EO) von 5 bis 7 besitzen.

10. Zusammensetzung gemäß einem der Ansprüche 1 bis 9, in welcher die durchschnittliche Gesamtlänge der Polyethoxylatreste in den Molekülen des Alkoholethoxylats (EO) folgende Bedingung erfüllt: EO ≥ C-6.

11. Zusammensetzung gemäß einem der Ansprüche 1 bis 10, welche ein gemischtes Alkoholethoxylat mit einer durchschnittlichen Alkylgesamtkettenlänge (C) von 10,75 bis 11,25 und einer gesamten durchschnittlichen Ethylenoxidkettenlänge (EO) von 5 bis 8 besitzt.

12. Zusammensetzung gemäß einem der Ansprüche 1 bis 11, wobei die Gewichtsverhältnisse der Gruppen R1 und R2, basierend auf den jeweiligen Alkoholen, in den Bereichen von 50:50 bis 85:15 liegen.

13. Zusammensetzung gemäß Anspruch 12, wobei die Gewichtsverhältnisse der Gruppen R1 und R2, basierend auf den jeweiligen Alkoholen, in den Bereichen von 60:40 bis 85:15 liegen.

14. Zusammensetzung gemäß Anspruch 13, wobei die Gewichtsverhältnisse der Gruppen R1 und R2, basierend auf den jeweiligen Alkoholen, in den Bereichen von 70:30 bis 80:20 liegen.

15. Zusammensetzung gemäß Anspruch 14, wobei die Gewichtsverhältnisse der Gruppen R1 und R2, basierend auf den jeweiligen Alkoholen, ungefähr 75:25 beträgt.

16. Verfahren zum Herstellen einer Zusammensetzung gemäß einem der Ansprüche 1 bis 15, welches folgendes einschließt: Ethoxylieren eines gemischten Alkohols mit einer durchschnittlichen Alkylkettenlänge wie in einem der Ansprüche 1 bis 15 definiert, um so ein gemischtes Alkoholethoxylat mit einer durchschnittlichen Zahl von EO-Einheiten von 4,5 bis 8 auszubilden.

17. Waschmittelformulierung einschließlich einer Alkoholethoxylatzusammensetzung gemäß einem der Ansprüche 1 bis 15.

18. Waschmittelformulierung gemäß Anspruch 17 in der Form fester Waschmittelkörner, einer wässrigen flüssigen Formulierung oder nicht wässriger flüssiger Formulierungen.

19. Waschmittelformulierung gemäß entweder Anspruch 17 oder Anspruch 18 in der Form eines Mehrstückpackungssystems.

20. Verfahren zur Wäschereinigung unter Verwendung einer Formulierung als Reinigungsmittel gemäß einem der Ansprüche 17 bis 19.

21. Verwendung von Alkoholethoxylatzusammensetzungen gemäß einem der Ansprüche 1 bis 15 oder 17 bis 19 als Reinigungsmittel bei der Wäschereinigung.

22. Harte Oberflächenreinigerformulierung einschließlich einer Alkoholethoxylatzusammensetzung gemäß einem der Ansprüche 1 bis 15.

23. Harte Oberflächenreinigerformulierung gemäß Anspruch 22 in der Form eines Mehrstückpackungssystems.

24. Verfahren zum Reinigen harter Oberflächen unter Verwendung einer Formulierung einschließlich einer Alkoholethoxylatzusammensetzung gemäß einem der Ansprüche 1 bis 15, 22 und 23.

25. Verwendung von Alkoholethoxylatzusammensetzungen gemäß einem der Ansprüche 1 bis 15 als Reinigungsmittel bei der Reinigung von harten Oberflächen.

26. Faser-, Garn- und Stoffreinigungsformulierungen einschließlich der Alkoholethoxylatzusammensetzung gemäß einem der Ansprüche 1 bis 15.

27. Verfahren zum Reinigen von Fasern, Garnen und Stoffen unter Verwendung von Formulierungen einschließlich der Alkoholethoxylatzusammensetzung gemäß einem der Ansprüche 1 bis 15.

28. Verwendung einer Alkoholethoxylatzusammensetzung gemäß einem der Ansprüche 1 bis 15 als ein oberflächenaktives Mittel bei der Faser-, Garn- oder Stoffreinigung.

29. Emulsionsformulierungen einschließlich der Alkoholethoxylatzusammensetzung gemäß einem der Ansprüche 1 bis 15.

30. Verfahren zum Emulgieren eines Öls in Wasser unter Verwendung einer Alkoholethoxylatzusammensetzung gemäß einem der Ansprüche 1 bis 15 als ein Emulgierungsmittel.

31. Verfahren gemäß Anspruch 30, wobei das Öl einen HLB-Wert in dem Bereich von 10 bis 14 aufweist.

32. Verwendung einer Alkoholethoxylatzusammensetzung gemäß einem der Ansprüche 1 bis 15 als ein Emulgierungsmittel zum Herstellen von Öl-in-Wasser-Emulsionen.

## Revendications

1. Composition tensioactive d'éthoxylats d'alcools des composés de formule (la) :
HO.(EO)ₙ₁.R¹ (Ia)
et de formule (Ib) :
HO.(EO)ₙ₂.R² (Ib)
dans lesquelles :
EO est un résidu d'oxyde d'éthylène (-CH₂-CH₂-O-) ;
n1 est de 3 à 8 ; et
n2 est de 4,5 à 9 ;
R¹ est un groupe alkyle choisi parmi des groupes alkyles ayant de 8 à 12 atomes de carbone et ayant un nombre moyen d'atomes de carbone de 9 jusqu'à moins de 12 ; et
R² est un groupe alkyle choisi parmi des groupes alkyles ayant de 12 à 16 atomes de carbone et ayant un nombre moyen d'atomes de carbone de 12 à 15 ;
selon des proportions pondérales telles que le nombre moyen global d'atomes de carbonc (C) dans les groupes alkyles R¹ et R² est de 10,5 à 12 et que le nombre moyen global d'unités EO (EO) est de 4,5 à 8.

2. Composition tensioactive d'éthoxylats d'alcools dans laquelle les éthoxylats d'alcools ont la formule (II) :
HO.(EO)ₙ₃.R³ (II)
dans laquelle :
EO est un résidu d'oxyde d'éthylène (-CH₂-CH₂-O-) ;
R³ est un groupe alkyle provenant de deux populations R^{3a} et R^{3b},
où :
dans la population R^{3a}, les groupes alkyles sont choisis parmi des groupes alkyles ayant de 8 à 12 atomes de carbone et ont un nombre moyen d'atomes de carbone de 9 jusqu'à moins de 12 ; et
dans la population R^{3b}, les groupes alkyles sont choisis parmi des groupes alkyles ayant de 12 à 16 atomes de carbone et ont un nombre moyen d'atomes de carbone de 12 à 15 ;
n3 provient de deux populations n3^{a} et n3^{b}, correspondant aux deux populations R^{3a} et R^{3b} respectivement, où n3^{a} est de 3 à 8 et n3^{b} est de 4,5 à 9 ;
où les proportions pondérales des deux populations sont telles que le nombre moyen global d'atomes de carbone (C) dans les groupes alkyles de R³ est de 10,5 à 12 et que la valeur moyenne global de n3 est de 4,5 à 8.

3. Composition tensioactive comprenant un éthoxylat d'un mélange d'alcools de formule (III)
HO.(EO)ₙ₄.R⁴ (III)
dans laquelle :
EO est un résidu d'oxyde d'éthylène (-CH₂-CH₂-O-) ;
n4 est de 4,5 à 8 ;
R⁴ est constitué de résidus d'un mélange d'alcools tels que:
une première partie des groupes alkyles est choisie parmi des groupes alkyles ayant de 8 à 12 atomes de carbone et ayant un nombre moyen d'atomes de carbone de 9 jusqu'à moins de 12 ; et une seconde partie des groupes alkyles est choisie parmi des groupes alkyles ayant de 12 à 16 atomes de carbone et ayant un nombre moyen d'atomes de carbone de 12 à 15 ; les alcools respectifs étant présents dans le mélange d'alcools dans des proportions pondérales telles que le nombre moyen global d'atomes de carbone (C) dans les groupes alkyles de R⁴ est de 10,5 à 12.

4. Composition suivant l'une quelconque des revendications 1 à 3, dans laquelle le nombre moyen global d'atomes de carbone (C) dans les résidus alkyles de la composition est de 10,5 à 11,5.

5. Composition suivant la revendication 4, dans laquelle le nombre moyen global d'atomes de carbone (C) est de 10,75 à 11,25.

6. Composition suivant l'une quelconque des revendications 1 à 5, dans laquelle l'alcool ayant une longueur moyenne de chaîne de carbone inférieure à 12 a une longueur moyenne de chaîne de carbone d'environ 9 à environ 11.

7. Composition suivant l'une quelconque des revendications 1 à 6, dans laquelle l'alcool ayant une longueur moyenne de chaîne de carbone de 12 ou plus, a une longueur moyenne de chaîne de carbone de 12 à 14,5.

8. Composition suivant l'une quelconque des revendications 1 à 7, dans laquelle la proportion des groupes alkyles ayant une longueur de chaîne qui n'est pas comprise dans les gammes indiquées pour les deux types de groupes alkyles est inférieure à 10% en poids (sur la base des alcools correspondants).

9. Composition suivant l'une quelconque des revendications 1 à 8, dans laquelle les résidus de polyéthoxylats présents dans les molécules de la composition d'éthoxylats d'alcools a une longueur moyenne globale (EO) de 5 à 7.

10. Composition suivant l'une quelconque des revendications 1 à 9, dans laquelle la longueur moyenne globale des résidus de polyéthoxylats présents dans les molécules d'éthoxylats d'alcools (EO) satisfait à la relation EO C - 6.

11. Composition suivant l'une quelconque des revendications 1 à 10, qui est un éthoxylat d'un mélange d'alcools ayant une longueur moyenne globale de chaîne des groupes alkyles (C) de 10,75 à 11,25 et une longueur moyenne globale des chaînes d'oxyde d'éthylène (EO) de 5 à 8.

12. Composition suivant l'une quelconque des revendications 1 à 11, dans laquelle les rapports pondéraux des groupes R¹ et R² sur la base des alcools respectifs sont compris dans les gammes de 50:50 à 85:15.

13. Composition suivant la revendication 12, dans laquelle les rapports pondéraux des groupes R¹ et R² sur la base des alcools respectifs sont compris dans les gammes de 60:40 à 85:15.

14. Composition suivant la revendication 13, dans laquelle les rapports pondéraux des groupes R¹ et R² sur la base des alcools respectifs sont compris dans les gammes de 70:30 à 80:20.

15. Composition suivant la revendication 14, dans laquelle le rapport pondéral des groupes R¹ et R² sur la base des alcools respectifs est d'environ 75:25.

16. Procédé de préparation d'une composition suivant l'une quelconque des revendications 1 à 15, qui comprend l'éthoxylation d'un mélange d'alcools ayant une longueur moyenne de chaîne des groupes alkyles telle que définie dans l'une quelconque des revendication 1 à 15 de façon à former un éthoxylat d'un mélange d'alcools ayant un nombre moyen d'unités EO de 4,5 à 8.

17. Formulation de détergent pour le lavage du linge comprenant une composition d'éthoxylats d'alcools suivant l'une quelconque des revendications 1 à 15.

18. Formulation de détergent pour le lavage du linge suivant la revendication 17, sous la forme de granulés de détergent solide, d'une formulation de liquide aqueux ou de formulations de liquides non-aqueux.

19. Formulation de détergent pour le lavage du linge suivant l'une ou l'autre des revendications 17 ou 18, sous la forme d'un système à plusieurs conditionnements.

20. Procédé de nettoyage du linge avec, en tant que détergent, une formulation suivant l'une quelconque des revendications 17 à 19.

21. Utilisation de compositions d'éthoxylats d'alcools suivant l'une quelconque des revendications 1 à 15, ou 17 à 19 en tant que détergents pour le lavage du linge.

22. Formulation d'agent de nettoyage de surfaces dures comprenant une composition d'éthoxylats d'alcools suivant l'une quelconque des revendications 1 à 15.

23. Formulation d'agent de nettoyage de surfaces dures suivant la revendication 22, sous la forme d'un système à plusieurs conditionnements.

24. Procédé de nettoyage de surfaces dures avec une formulation comprenant une composition d'éthoxylats d'alcools suivant l'une quelconque des revendications 1 à 15, 22 ou 23.

25. Utilisation de compositions d'éthoxylats d'alcools suivant l'une quelconque des revendications 1 à 15, en tant que détergents pour le nettoyage de surfaces dures.

26. Formulations pour le dégraissage de fibres, fils et tissus comprenant la composition d'éthoxylats d'alcools suivant l'une quelconque des revendications 1 à 15.

27. Procédé de dégraissage de fibres, fils et tissus avec des formulations comprenant la composition d'éthoxylats d'alcools suivant l'une quelconque des revendications 1 à 15.

28. Utilisation d'une composition d'éthoxylats d'alcools suivant l'une quelconque des revendications 1 à 15, en tant que détergent dans le dégraissage de fibres, fils ou tissus.

29. Formulations pour émulsion comprenant les compositions d'éthoxylats d'alcools suivant l'une quelconque des revendications 1 à 15.

30. Procédé d'émulsification d'une huile dans l'eau avec une composition d'éthoxylats d'alcools suivant l'une quelconque des revendications 1 à 15 en tant qu'émulsifiant.

31. Procédé suivant la revendication 30, dans lequel l'huile a une exigence HLB comprise dans la gamme de 10 à 14.

32. Utilisation d'une composition d'éthoxylats d'alcools suivant l'une quelconque des revendications 1 à 15 en tant qu'émulsifiant pour préparer des émulsions d'huile dans l'eau.
